# EUROPEAN PATENT APPLICATION

(11) **EP 3 229 016 A1**
(43) Date of publication of application: **11.10.2017**
(21) Application number: 15865044.0
(22) Date of filing: 03.12.2015
(51) Int. Cl.: G01N 27/00, G01N 31/00

(54) **DEVICE AND METHOD USED FOR REMOVING INORGANIC CARBON, AND APPARATUS AND METHOD USED FOR DETECTING TOTAL ORGANIC CARBON**

(30) Priority: 03.12.2014 CN 201410724329
(71) Applicant: General Electric Company, Schenectady, NY 12345 (US)
(72) Inventor: DENG, Shouquan, Shanghai 201203 (CN)
(74) Representative: Foster, Christopher Michael
(86) International application number: PCT/CN2015/000844
(87) International publication number: WO 2016/086507

(57) **Abstract**

Provided is a device (100, 200, 300) used for removing inorganic carbon, comprising: a first space (101, 201, 301), used for receiving a first water flow (102, 202, 302) containing inorganic carbon of a first concentration, and for providing carbon dioxide (103, 203, 303) converted from said inorganic carbon, and an analysis flow (104, 204, 304) containing inorganic carbon of a second concentration, said second concentration being lower than said first concentration; and a second space (105, 205, 305), used for receiving a second water flow (106, 206, 306) and absorbing the carbon dioxide (103, 203, 303) coming from said first space (101, 201, 301). Also disclosed are a method used for removing inorganic carbon, and an apparatus and method used for detecting total organic carbon.

## Description

### FIELD

The present invention relates to device and method for removing inorganic carbon and apparatus and method for measuring total organic carbon.

### BACKGROUND

Currently, inorganic carbon is commonly removed from water by converting the inorganic carbon into carbon dioxide and vacuuming. However, with such approaches, other substances such as volatile organics in the water are usually pumped out together, which is not desired.

Especially in the case of measuring total organic carbon, when the volatile organics are pumped away, the measured total organic carbon is actually Non-Purgeable Organic Carbon, which cannot thoroughly reflect the total content of the organic carbon in the water.

Thus, there is a need to provide device and method for removing inorganic carbon and apparatus and method for measuring total organic carbon.

### SUMMARY

An objective of the present invention is to provide device and method for removing inorganic carbon and apparatus and method for measuring total organic carbon.

In an aspect, embodiments of the present invention relate to a device for removing inorganic carbon, comprising: a first compartment for receiving a first aqueous stream having a first concentration of inorganic carbon and providing carbon dioxide converted from the inorganic carbon and an analysis stream having a second concentration of inorganic carbon, the second concentration being lower than the first concentration; and a second compartment for receiving a second aqueous stream and absorbing the carbon dioxide from the first compartment.

In another aspect, embodiments of the present invention relate to an apparatus for measuring total organic carbon, comprising: the device for removing inorganic carbon relating to the embodiments of the present invention; and a total organic carbon analyzer for receiving the analysis stream and measuring the total organic carbon of the analysis stream.

In a further aspect, embodiments of the present invention relates to a method for removing inorganic carbon, comprising: receiving in a first compartment a first aqueous stream having a first concentration of inorganic carbon and providing carbon dioxide converted from the inorganic carbon and an analysis stream having a second concentration of inorganic carbon, the second concentration being lower than the first concentration; and receiving a second aqueous stream and absorbing in a second compartment the carbon dioxide from the first compartment.

In a further aspect, embodiments of the present invention relate to a method for measuring total organic carbon, comprising: the method for removing inorganic carbon relating to embodiments of the present invention; and measuring the total organic carbon of the analysis stream using a total organic carbon analyzer........

### BRIEF DESCRIPTION OF THE DRAWINGS

Features, aspects, and advantages of the present invention will become better understood when the following detailed description is read with reference to the accompanying drawings, wherein:

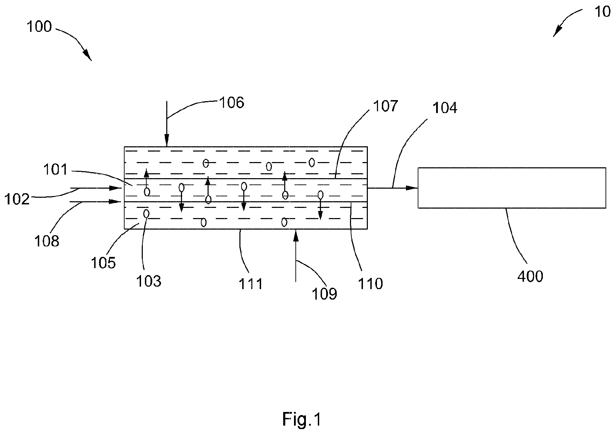

FIGS. 1-3 illustrate schematic views of apparatuses for measuring total organic carbon according to some embodiments of the present invention.

### DETAILED DESCRIPTION

Unless defined otherwise, all the scientific or technical terms used herein should have the same meanings as commonly understood by one of ordinary skilled in the art to which the present invention belongs. The terms "first", "second" and the like used in the present invention do not mean any sequential order, number or importance, but are only used for distinguishing from one another. The terms "comprising", "including", "having", "containing" and the like used in the present invention mean that, in addition to the items listed thereafter and equivalents thereof, other items may also be encompassed therein.

The approximate expression in the present invention is used to define the quantity, indicating that the present invention is not limited to the exact quantity, but encompass modifications that are close to the quality, acceptable and do not result in changes of corresponding basic functions thereof. Accordingly, defining a quantity by "about", "approximately" or the like means that the present invention is not limited to the exact numerical value. In some embodiments, approximate expression may correspond to the precision of the instrument that measures numerical values.

Unless explicitly indicated otherwise, in the Description and claims, the singular or plural form of all items is not limited. Unless explicitly stated otherwise, the term "or" does not mean exclusion, but means there is at least one of the mentioned items (such as composition), including situations that there may be combinations of the mentioned items.

Reference to "some embodiments" in the Description of the present invention means that a particular element (e.g. a feature, a structure and/or a characteristic) related to the present invention is included in at least one embodiment of the Description and may or may not be present within other embodiments. Furthermore, it is to be understood that elements of the present invention may be combined in any suitable manner.

Referring to figures 1, 2, and 3, some embodiments of the present invention relates to devices 100, 200, 300 for removing inorganic carbon, comprising: a first compartment 101, 201, 301 for receiving a first aqueous stream 102, 202, 302 having a first concentration of inorganic carbon and providing carbon dioxide 103, 203, 303 converted from the inorganic carbon and an analysis stream 104, 204, 304 having a second concentration of inorganic carbon, the second concentration being lower than the first concentration; and a second compartment 105, 205, 305 for receiving a second aqueous stream 106, 206, 306 and absorbing the carbon dioxide 103, 203, 303 from the first compartment 101, 201, 301.

In some embodiments, as shown in figure 1, the device 100 comprises an inner tube 110 defining therein a first compartment 101 and an outer tube 111 nested outside the inner tube 110, the second compartment 105 being located between the inner tube 110 and the outer tube 111 and surrounding the first compartment 101. The tube wall 107 of the inner tube 110 is a separator which separates the first aqueous stream 102 from the second aqueous stream 106 and allows the carbon dioxide 103 to pass through.

The material, shape, and configuration for the inner tube and the outer tube may be the same or substantially different. In figure 1, both of the inner tube 110 and the outer tube 111 extends linearly. In some embodiments, both of the inner tube and the outer tube meander. In some embodiments, one of the inner tube and the outer tube is a straight tube and the other is a curved tube. In some embodiments, the inner tube and the outer tube may be straight in part and curved in part.

In some embodiments, the first compartment surrounds the second compartment. In some embodiments, the first compartment overlaps the second compartment in part.

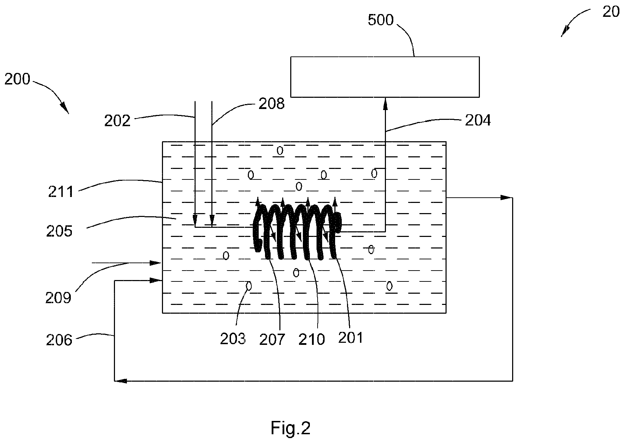

In some embodiments, as shown in figure 2, the device 200 comprises a tube 210 which defines a first compartment 201 therein and extends in a curved manner and a container 211 which contains the tube 210 therein and defines a second compartment 205 therebetween. The first compartment 201 is located within the tube 210 and is surrounded by the second compartment 205. The tube wall 207 of the tube 210 is a separator which separates the first aqueous stream 202 from the second aqueous stream 206 and allows the carbon dioxide 203 to pass through.

The separators 107, 207 may be made of any material that allow gas to pass through but does not allow liquid to pass through. In some embodiments, the separators 107, 207 comprise Polypropylene, polytetrafluoroethylene, or polyethylene. In some embodiments, the inner tube 110 and the tube 210 are Teflon ^{®} tubes.

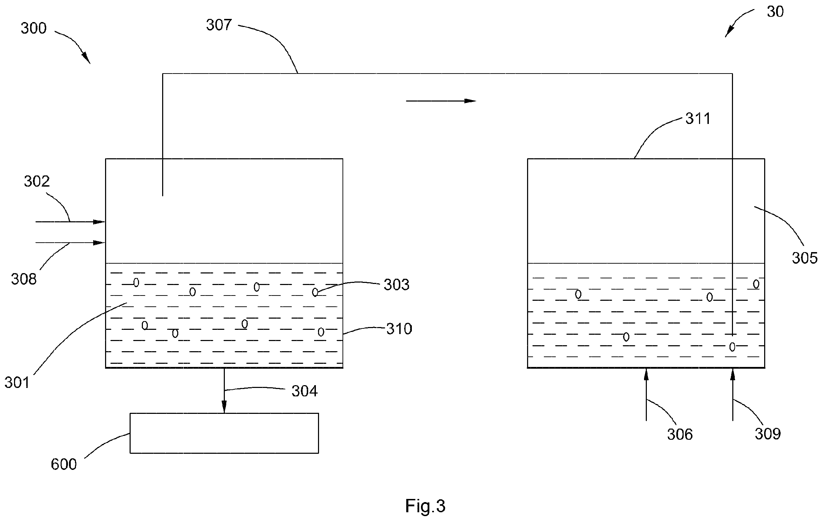

In some embodiments, as shown in figure 3, the device 300 comprises a first container 310 and a second container 311 which are separated by a certain distance from each other, define a first compartment 301 and a second compartment 305 therein respectively, and are connected together by a conduit 307. The conduit 307 transports the dioxide 303 from the first compartment 301 to the second compartment 305.

The first aqueous streams 102, 202, 302 are aqueous streams in which the inorganic carbon therein needs to be removed. In addition to water and inorganic carbon, the first aqueous streams 102, 202, 302 may or may not comprise other substances. In some embodiments, the first aqueous streams 102, 202, 302 contain water, inorganic carbon, volatile organics and non-volatile organics.

In the first compartments 101, 201, 301, the inorganic carbons in the first aqueous streams 102, 202, 302 are converted into dioxides 103, 203, 303 in a suitable manner. In some embodiments, reactants 108, 208, 308 are provided to the first compartments 101, 201, 301 to convert the inorganic carbons into the dioxides 103, 203, 303. In some embodiments, the reactants 108, 208, 308 are acid compounds, such as phosphoric acid, hydrochloric acid, sulfuric acid or any combination thereof.

Generation of dioxides 103, 203, 303 creates gas pressure difference between the first compartments 101, 201, 301 and the second compartments 105, 205, 305, such that the dioxides enter from the first compartments 101, 201, 301 through the separators 107, 207 and the conduit 307 into the second compartments 105, 205, 305 and are absorbed by the second compartments 105, 205, 305, which results in generation of the analysis streams 104, 204, 304 having a concentration of inorganic carbon lower than that of the first aqueous streams 102, 202, 302.

In some embodiments, the second aqueous streams 106, 206, 306 help to absorb the dioxides 103, 203, 303. In some embodiments, the second aqueous streams 106, 206, 306 are the same as the first aqueous streams 102, 202, 302, such that the gas pressure difference between the first compartments 101, 201, 301 and the second compartments 105, 205, 305 mainly results from generation of dioxides 103, 203, 303 in the first compartments 101, 201, 301, and gas that flows between the first compartments 101, 201, 301 and the second compartments 105, 205, 305 is mainly the carbon dioxides 103, 203, 303.

In some embodiments, reactants 109, 209, 309 are provided to the second compartments 105, 205, 305 to assist in absorbing the dioxides 103, 203, 303. In some embodiments, the reactants 109, 209, 309 comprise alkali compound, such as sodium hydroxide, potassium hydroxide, lithium hydroxide or any combination thereof. In some embodiments, the reactants 109, 209, 309 comprise complexes that may prevent sedimentation, such as ethylenediaminetetraacetic acid, ethylenediamine tetraacetate (disodium ethylenediamine tetraacetate, tetrasodium ethylenediamine tetraacetate, or the like), Ethylene Diamine Tetra (Methylene Phosphonic Acid) Sodium, Nitrilotriacetic acid sodium, trimethylene amine phosphates, diethylenetriamine pentacarboxylic acid salts, Diethylene Triamine Penta (Methylene Phosphonic Acid) salt, citric acid, or any combination thereof.

In some embodiments, the devices 100, 200, 300 comprise a stirring element (not shown) that may stir in the first compartments 101, 201, 301 or the second compartments 105, 205, 305 to facilitate generation or absorption of the carbon dioxides.

After removing the inorganic carbons with the device and method relating to the embodiments of the present invention, the total organic carbon of the analysis streams 104, 204, 304 may be measured by any method and device for measuring total organic carbon. Some embodiments of the present invention relate to apparatuses 10, 20, 30 and method for measuring total organic carbon. In some embodiments, the apparatuses 10, 20, 30 comprise the devices 100, 200, 300 and the total organic carbon analyzers 400, 500, 600. The total organic carbon analyzers 400, 500, 600 may be any total organic carbon analyzer or sensor, such as the total organic carbon analyzer of Sievers® M series (e.g. M9 total organic carbon analyzer, M5310 total organic carbon analyzer) or CheckPoint sensor from GE Analytical Instruments Co., Ltd. of Boulder, State of Colorado.

In some embodiments, the total organic carbon analyzers 400, 500, 600 receive the analysis streams 104, 204, 304, and, after steps of acidizing, oxidizing, measuring carbon dioxide generated from total carbon and total inorganic carbon with a embrane conductivity detector or the like, calculating difference between the total carbon and the total inorganic carbon to obtain the total organic carbon, output the total inorganic carbon value, the total carbon value and the total organic carbon value of the analysis streams 104, 204, 304. In some embodiments, when measuring the total carbon with the total organic carbon analyzers 400, 500, 600, ultraviolet light may be used to oxidize, or oxidizing agent such as persulfate may be added, or combustion oxidation with high temperature and catalytic may be used.

The device and method for removing inorganic carbon relating to embodiments of the present invention utilize the pressure difference between the first compartment and the second compartment and have the carbon dioxide converted from the inorganic carbon of the first compartment be absorbed in the second compartment, so as to realize removal of the inorganic carbon. In some embodiments, the second aqueous stream facilitates absorption of the carbon dioxide. In some embodiments, the concentration of the volatile organic of the first compartment is the same as that of the second compartment, such that the volatile organics would have no substantial loss when removing the inorganics. The apparatus and method for measuring total organic carbon relating to embodiments of the present invention can measure the total organic carbon more precisely.

### EXPERIMENTAL EXAMPLES

The following experimental examples may provide references for a person with ordinary skills in the art in practicing the present invention. Extent of the claims are not limited by these examples.

Chloroform solution with 400 ppb (parts per billion) of carbon is prepared by mixing 100% chloroform into water.

### Comparative example 1

A stream of the above prepared chloroform solution is introduced into a total organic carbon analyzer with a flow rate of 0.5 milliliter per minute without removing the inorganic carbon, where it is acidized by adding phosphoric acid with 6 mol per liter, oxidized with ultraviolet light, measured with a membrane conductivity detector, and the resultant total organic carbon value is 400 ppb, inorganic carbon value is 704 ppb, which are the measurement baseline values of the total organic carbon and inorganic carbon for the prepared chloroform solution.

### Comparative example 2

Another stream of the above prepared chloroform solution is added with phosphoric acid of 6 mol per liter and is purged of inorganic carbon by vacuuming, and is then introduced into the same total organic carbon analyzer as comparative example 1 with a flow rate of 0.5 milliliter per minute. The resultant total organic carbon value is 158 ppb, inorganic carbon value is 16 ppb. It can be seen that, in comparison with the measurement baseline values of comparative example 1, more than half of the organic carbon (volatile organics) are lost when purging inorganic carbon in the chloroform solution by vacuuming, and the measured total organic carbon is far less than the measurement baseline value.

### Example 1

A Teflon ^{®}AF curved tube is placed in a container (a glass beaker), so that the device for removing inorganic carbon as shown in figure 2 is implemented.

A stream of the above prepared chloroform solution is introduced into the first compartment in the curved tube and the second compartment outside the curved tube and inside the container with a flow rate of 0.5 microlitre per minute. Phosphoric acid of 6 mol per liter is added into the first compartment, sodium hydroxide which provides the chloroform solution with pH>7 and ethylenediaminetetraacetic acid of 0.01 mol per litre are provided to the second compartment.

The analysis stream from the first compartment is introduced into the same total organic carbon analyzer as comparative example 1 with a flow rate of 0.5 milliliter per minute. The resultant total organic carbon value is 375 ppb, inorganic carbon value is 327 ppb. It can be seen that, in comparison with the measurement baseline values of comparative example 1, while inorganic carbon in the chloroform solution is reduced by more than half, the reduction of the organic carbon is negligible, and the measured total organic carbon is more reliable and accurate.

Although the present invention has been set forth in connection with specific embodiments, the person skilled in the art shall be understood that many modifications and variations may be made to the present invention. Therefore, it should be recognized that the intention of the claims is to cover all these modifications and variations within the real concept and range of the present invention.

## Claims

1. A device for removing inorganic carbon, comprising:
a first compartment for receiving a first aqueous stream having a first concentration of inorganic carbon and providing carbon dioxide converted from the inorganic carbon and an analysis stream having a second concentration of inorganic carbon, the second concentration being lower than the first concentration; and
a second compartment for receiving a second aqueous stream and absorbing the carbon dioxide from the first compartment.

2. The device of claim 1, wherein the first and the second compartments comprise a same concentration of volatile organic compound.

3. The device of claim 1, comprising a separator for separating the first aqueous stream from the second aqueous stream and allowing the carbon dioxide to pass through.

4. The device of claim 1, comprising a conduit for transporting the carbon dioxide from the first compartment to the second compartment.

5. An apparatus for measuring total organic carbon, comprising:
the device of any of claims 1-4; and
a total organic carbon analyzer for receiving the analysis stream and measuring the total organic carbon of the analysis stream.

6. A method for removing inorganic carbon, comprising:
receiving in a first compartment a first aqueous stream having a first concentration of inorganic carbon and providing carbon dioxide converted from the inorganic carbon and an analysis stream having a second concentration of inorganic carbon, the second concentration being lower than the first concentration; and
receiving a second aqueous stream and absorbing in a second compartment the carbon dioxide from the first compartment.

7. The method of claim 6, wherein the first and the second compartments comprise a same concentration of volatile organic compound.

8. The method of claim 6, comprising: providing a reagent to the first compartment to convert the inorganic carbon to the carbon dioxide.

9. The method of claim 6, comprising: providing a reagent to the second compartment to absorb the carbon dioxide.

10. A method for measuring total organic carbon, comprising:
the method for removing inorganic carbon of any of claims 6-9; and
measuring the total organic carbon of the analysis stream using a total organic carbon analyzer.
